# EUROPÄISCHE PATENTANMELDUNG

(11) **EP 3 718 519 A1**
(43) Veröffentlichungstag der Anmeldung: **07.10.2020**
(21) Anmeldenummer: 20155933.3
(22) Anmeldetag: 06.02.2020
(51) Int. Cl.: A61F 7/00, A61F 7/02

(54) **SCHUTZHÜLLE FÜR PASSIVE WÄRME- UND KÄLTETRÄGER**

(30) Priorität: 04.04.2019 CH 4592019
(71) Anmelder: Fallegger, Angela, 6055 Alpnach (CH); Aggeler, Patrick, 6055 Alpnach (CH)
(72) Erfinder: Fallegger, Angela, 6055 Alpnach (CH); Aggeler, Patrick, 6055 Alpnach (CH)
(74) Vertreter: Koelliker, Robert

(57) **Zusammenfassung**

Die vorliegende Erfindung bezieht sich auf eine Schutzhülle (1) für passive Wärme- und Kälteträger (2) umfassend mindestens eine Schicht A und eine Schicht B, wobei die Schicht A einen grösseren Wärmedurchgangskoeffizienten (U-Wert) aufweist als die Schicht B. Die Schutzhülle (1) umfasst bevorzugt eine weitere Schicht C. Mit der Schutzhülle (1) umfassend lediglich die Schichten A, B und C ist es auf einfache Art und Weise möglich, den in der Schutzhülle (1) angeordneten gewärmten Wärmeträger (2a) oder gekühlten Kälteträger (2b) mit bis zu vier unterschiedlich isolierenden Schichten optimal auf das zu wärmende oder kühlende Substrat einzustellen.

Beansprucht wird auch ein Verfahren zum Wärmen oder zum Kühlen von Substraten mit der erfindungsgemässen Schutzhülle (1) sowie die Verwendung der Schutzhülle (1).

## Beschreibung

Die vorliegende Erfindung bezieht sich auf eine Schutzhülle für passive Wärme- und Kälteträger, ein Verfahren zum Wärmen oder zum Kühlen von Substraten mit der Schutzhülle sowie die Verwendung der Schutzhülle.

Wärmen und Kühlen von Körperstellen gehören seit langem zu den Hausmitteln, um leichtere Verletzungen zu behandeln und das Wohlempfinden zu erhöhen.

Beim Wärmen und Kühlen von Körperstellen wird zwischen aktivem und passivem Wärmen/Kühlen unterschieden. Beim aktiven Wärmen/Kühlen wird während dem Wärmen/Kühlen mit Hilfe einer zusätzlichen Einheit und in der Regel mit Stromanschluss Energie zu- oder abgeführt, beispielsweise bei elektrischen Heizkissen. Dadurch kann die Temperatur des Wärme-/ Kühlelements während dem Wärmen/Kühlen konstant gehalten werden. Beim passiven Wärmen/Kühlen wird während dem Wärmen/Kühlen keine externe Verbindung benötigt, sondern das Wärme-/Kühlelement wird vorgängig gewärmt oder gekühlt. Während dem Wärmen/Kühlen gleicht sich die Temperatur des Wärme-/Kühlelements der Umgebungstemperatur an. Somit ist die Wärme-/Kühldauer zeitlich begrenzt. Da beim passiven Wärmen/Kühlen jedoch keine zusätzliche Apparatur benötigt wird, ist diese Methode ortsunabhängig und der Patient mit dem Wärme-/Kühlelement ist mobil.

Zum passiven Wärmen werden traditionell u.a. kleine Säcke mit Kernen von Früchten eingesetzt, die zuvor in einem Ofen oder Mikrowellengerät erwärmt worden sind. Und zum passiven Kühlen werden Eis, Schnee und oft auch Kühlelemente, auch Kühlakkus genannt, eingesetzt, welche mit einer Kühlflüssigkeit oder einem Kühlgel gefüllt sind. Alle diese Materialien besitzen eine grosse Wärmekapazität und können dadurch über eine längere Zeit wärmend oder kühlend wirken.

Um die Wärmedauer von passiven Wärmeträgern zu verlängern, werden diese oft stärker erwärmt. Damit sie beim Wärmen der Körperstellen jedoch keine Verletzungen hervorrufen, werden die Wärmeträger zuvor beispielsweise in ein Tuch eingewickelt. Lässt die Wärmewirkung nach, muss das Tuch umständlich neu um den Wärmeträger gewickelt werden, sodass die Wärmeisolation geringer ist, aber nicht so gering, dass der Wärmeträger auf der Haut als zu heiss empfunden wird.

Auch bei passiven Kälteträgern wird in der Regel eine Trennschicht - beispielsweise ein Tuch oder Kleidungsstück - zwischen den Kälteträger und die zu kühlende Körperstelle angeordnet. Nach einer gewissen Zeit wird die Dicke der Trennschicht reduziert, um die Kühlwirkung weiterhin aufrecht zu halten.

Werden isolierenden Trennschichten wie Tücher eingesetzt, muss sowohl beim Wärmen wie beim Kühlen darauf geachtet werden, dass die Trennschicht korrekt angeordnet bleibt, um keine Verletzungen oder eine zu starke Isolation durch den Wärme- oder Kälteträger hervorzurufen. Dies ist oft eine Herausforderung, beispielsweise wenn sich der Patient - beispielsweise Kinder - bewegen. Auch ist es oft mühsam die Wärme-/ Kühlphase der Trennschicht so anzupassen, dass die isolierende Wirkung gerade gut, d.h. nicht zu stark, aber auch nicht zu schwach ist.

Daher ist es die Aufgabe der vorliegenden Erfindung, eine Trennschicht für Wärme- und Kälteträger bereitzustellen, welche variable, leicht einstellbare Wärme-isolierende Eigenschaften aufweist.

Die Aufgabe konnte überraschenderweise gelöst werden mit einer Schutzhülle (1) für passive Wärme- und Kälteträger (2), umfassend mindestens eine Schicht A und eine Schicht B, wobei die Schichten A und B an mindestens 2 Seiten zumindest teilweise aneinander befestigt sind, wobei die Schicht A einen grösseren Wärmedurchgangskoeffizienten, d.h. U-Wert, aufweist als die Schicht B.

Beansprucht wird auch ein Verfahren zum Wärmen oder zum Kühlen von Substraten mit der erfindungsgemässen Schutzhülle (1), wobei
- mindestens ein gewärmter passiver Wärmeträger (2a) oder ein gekühlter passiver Kälteträger (2b) zwischen die Schicht A und die Schicht B oder zwischen die Schicht B und die optionale Schicht C der Schutzhülle (1) gelegt wird, wodurch der in der Schutzhülle (1) angeordnete Wärme- oder Kälteträger (2) auf einer Seite mit einer Schicht oder zwei Schichten mit insgesamt einem grösseren Wärmedurchgangskoeffizienten und auf der anderen Seite mit einer Schicht oder zwei Schichten mit insgesamt einem geringerem Wärmedurchgangskoeffizienten bedeckt ist,
- die Schutzhülle (1) mit dem passiven Wärme- oder Kälteträger (2) so auf ein Substrat gelegt wird, dass die Schicht oder Schichten mit dem insgesamt geringeren Wärmedurchgangskoeffizienten der Schutzhülle (1) auf das Substrat zu liegen kommt, wobei das Substrat gegebenenfalls bedeckt sein kann,
- die Schutzhülle (1) mit dem Wärme- oder Kälteträger (2) eine Zeit lang auf dem Substrat belassen wird bis die Wärmwirkung des Wärmeträgers (2a) oder die Kühlwirkung des Kälteträgers (2b) nachlässt,
- anschliessend die Schutzhülle (1) mit dem Wärme- oder Kälteträger (2) gewendet wird, dass die Schicht oder Schichten der Schutzhülle (1) mit dem insgesamt grösseren Wärmedurchgangskoeffizienten auf das Substrat zu liegen kommt, und
- die Schutzhülle (1) mit dem Wärme- oder Kälteträger (2) eine Zeit lang auf dem Substrat belassen wird bis die Wärmwirkung des Wärmeträgers (2a) oder die Kühlwirkung des Kälteträgers (2b) weiter nachlässt,
wobei das Substrat, auf welches die Schutzhülle (1) mit dem Wärme- oder Kälteträger (2) gelegt wird, gegebenenfalls auch bedeckt sein kann, beispielsweise mit Verpackungsmaterial, einem Kleidungsstück oder einem Tuch.

Zudem wird auch die Verwendung der erfindungsgemässen Schutzhülle (1) und der erfindungsgemäss hergestellten Schutzhülle (1) zur Wärme- und/oder Kältebehandlung von Substraten wie Lebensmittel und/oder Körperstellen von lebenden Körpern von Menschen oder Tieren, insbesondere in Spitälern, medizinischen Praxen und/oder im privaten Bereich, beansprucht.

Die erfindungsgemässe Schutzhülle (1), das erfindungsgemässe Verfahren sowie die erfindungsgemässe Verwendung weisen überraschenderweise viele Vorteile auf. So kann ein stark aufgewärmter Wärmeträger (2a), auch Wärmebeutel genannt, oder ein stark gekühlter Kälteträger (2b), auch Kältebeutel genannt, in die Schutzhülle (1) gelegt werden. Dabei wird diese so auf das zu wärmende oder zu kühlende Substrat gelegt, dass zwischen dem Wärme- oder Kälteträger (2) die Schicht B mit dem kleineren Wärmedurchgangskoeffizienten, d.h. der stärker Wärme-isolierenden Schicht, liegt. Gleicht sich die Temperatur des Wärme- oder Kälteträgers (2) der Körpertemperatur an, kann die Schutzhülle (1) einfach mit dem darin liegenden Wärme- oder Kälteträger (2) gedreht werden, sodass die Schicht A mit dem grösseren Wärmedurchgangskoeffizienten, d.h. der schwächer Wärme-isolierenden Schicht, zwischen dem Wärme- oder Kälteträger (2) und dem Substrat zu liegen kommt. Dadurch fliesst mehr Wärme vom Träger (2) zum Substrat resp. vom Substrat zum Träger und die wärmende resp. kühlende Wirkung wird wieder erhöht.

Umfasst die Schutzhülle (1) eine optionale Schicht C, welche an mindestens einer Seite an der Schutzhülle (1) befestigt ist und einen Wärmedurchgangskoeffizienten aufweist, welcher beispielsweise mit dem Wärmedurchgangskoeffizienten der Schicht A oder dem Wärmedurchgangskoeffizienten der Schicht B identisch ist, ist es überraschenderweise auf einfache Art und Weise möglich, mittels lediglich drei Schichten A, B und C vier verschiedene Wärme-isolierende Schichtkombinationen der Schutzhülle (1) zu erhalten. Weist beispielsweise die Schicht A einen doppelt so grossen U-Wert auf wie die Schicht B, welcher auch etwa dem U-Wert der Schicht C entsprechen kann, und wird der Wärme- oder Kälteträger (2) zwischen die Schicht A und die Schichten B und C gelegt, isolieren die nebeneinander liegenden Schichten B (++) und C (++) zusammen etwa viermal (++++) so fest wie die Schicht A (+) alleine. Wird nun die Schutzhülle (1) so verändert, dass die Schicht C neben die Schicht A zu liegen kommt oder der Wärme- oder Kälteträger (2) zwischen die Schichten B und C angeordnet wird, isolieren die nebeneinander liegenden Schichten A und C resp. A und B etwa dreimal (+++) so viel und die Schicht B resp. die Schicht C etwa doppelt (++) so viel wie die Schicht A (+) allein. Dabei kann die erfindungsgemässe Schutzhülle (1) bei geeigneter Ausführung überraschenderweise auf einfache Art und Weise dergestalt verändert werden, ohne dass der Wärme- oder Kälteträger (2) zwingend herausgenommen und neu platziert werden muss.

Somit ist es mit der Schutzhülle (1) umfassend lediglich die Schichten A, B und C auf einfache Art und Weise möglich, den in der Schutzhülle (1) angeordneten gewärmten Wärmeträger (2a) oder gekühlten Kälteträger (2b) mit bis zu vier unterschiedlich isolierenden Schichten optimal auf das zu wärmende oder kühlende Substrat einzustellen.

### Die Schutzhülle (1)

Die erfindungsgemässe, erfindungsgemäss hergestellte und erfindungsgemäss verwendete Schutzhülle (1) ist insbesondere geeignet um passive Wärme- oder Kälteträger (2), auch Wärme- oder Kälteträger (2) genannt, aufzunehmen, um an lebenden Körpern eine Wärme- und/oder Kältebehandlung vorzunehmen.

Geeignete passive Wärmeträger (2a) zum passiven Wärmen können in einem Ofen, Mikrowellengerät und/oder in der Nähe eines Feuers aufgewärmt werden. Sie umfassen typischerweise eine äussere Schicht, welche mindestens ein Material mit einer erhöhten Wärmekapazität umfasst. Materialien für passive Wärmeträger (2a) eignen sich insbesondere Kerne von Steinobst wie von Steinfrüchten umfassend Pfirsich, Kirschen, Nektarinen, Aprikosen, Zwetschgen, Pflaumen und Mirabellen; und Kernobst umfassend Äpfel und Birnen. Zudem können Latentwärmespeicher, auch Phasenwechselmaterial oder PCM genannt, verwendet werden. Diese weisen einen Phasenübergang mit einer erhöhten Wärmkapazität auf, d.h. es braucht viel Energie, um einen festen Latentwärmespeicher zu schmelzen. Diese Energie wird dann beim Abkühlen wieder im Bereich der Schmelztemperatur frei. Geeignete, nicht-limitierende Latentwärmespeicher umfassen Paraffine mit einer Schmelztemperatur zwischen 50-100°C, bevorzugt zwischen 65°-85°C. Dem Fachmann sind solche passive Wärmeträger (2a) bekannt und sie sind im Handel erhältlich.

Geeignete passive Kälteträger (2b) zum passiven Kühlen können in einem Kühl- oder Gefrierschrank gekühlt werden oder liegen in gekühlter Form in der Natur - beispielsweise im Winter - vor. Sie umfassen typischerweise eine äussere Schicht, welche mindestens ein Material mit einer erhöhten Wärmekapazität und einen niedrigen Schmelztemperatur, beispielsweise zwischen 0°C und -30°C, umfasst. Materialien für passive Kälteträger (2b) eignen sich insbesondere Schnee, Eis, Kühlgele sowie Latentwärmespeicher. Diese werden zur Aktivierung gefroren. Bei deren Verwendung in der Schutzhülle (1) wird dann viel Energie von aussen benötigt, beispielsweise von dem zu kühlenden Substrat, um das gefrorene Latentwärmespeichermaterial, beispielsweise Paraffin, zu schmelzen. Bevorzugte, nicht-limitierende Latentwärmespeicher umfassen Paraffine mit einer Schmelztemperatur zwischen 0°C und -20°C. Dem Fachmann sind geeignete passive Kälteträger (2b) bekannt und sie sind im Handel erhältlich.

Die Schutzhülle (1) umfasst mindestens eine Schicht A und eine Schicht B, wobei die Schichten A und B an mindestens 2 Seiten zumindest teilweise aneinander befestigt sind. Dabei weist die Schicht A einen grösseren Wärmedurchgangskoeffizient (U-Wert) aufweist als die Schicht B, d.h. die Schicht A weist eine geringere Wärme/Kälte-isolierende Wirkung auf als die Schicht B.

In einer besonders bevorzugten Ausführungsform umfasst die Schutzhülle (1) eine weitere Schicht C, welche an mindestens einer Seite an der Schutzhülle (1) befestigt ist. Dabei weist die die Schicht C bevorzugt einen Wärmedurchgangskoeffizienten auf, welcher mit dem Wärmedurchgangskoeffizienten der Schicht A oder dem Wärmedurchgangskoeffizienten der Schicht B identisch ist.

Unter dem Begriff Wärmedurchgangskoeffizient, auch U-Wert genannt, versteht der Fachmann ein Koeffizient, welcher angibt, wieviel Wärme in Watt (W) pro Quadratmeter Fläche (m²) je Grad Temperaturdifferenz (K) von einer Seite zur anderen Seite des Materials fliesst. Der U-Wert wird somit in W/m²K angegeben und ist unabhängig von der jeweiligen Schichtdicke.

Geeignete, nicht-limitierende U-Werte der Schichten A, B und C der Schutzhülle (1) liegen typischerweise zwischen 1 bis 50 W/m²K.

Der Begriff "grösserer Wärmedurchgangskoeffizient" bedeutet, dass der Wärmedurchgangskoeffizient der Schicht mit dem grösseren Wärmedurchgangskoeffizienten, d.h. der Schicht A, mindestens 5%, bevorzugt mindestens 10%, insbesondere mindestens 25%, grösser ist als der Wärmedurchgangskoeffizient der Schicht mit dem geringeren, d.h. kleineren, Wärmedurchgangskoeffizienten, d.h. der Schicht B.

In einer bevorzugten Ausführungsform weist die Schicht B einen um einen Faktor 1.5 bis 5 kleineren Wärmedurchgangskoeffizienten auf als die Schicht A. Mit anderen Worten: Beträgt der Wärmedurchgangskoeffizient der Schicht A beispielsweise 10 W/m²K, so beträgt der Wärmedurchgangskoeffizient der Schicht B bevorzugt zwischen 6.7 und 2 W/m²K.

In einer anderen bevorzugten Ausführungsform weist die Schicht A einen um einen Faktor 1.5 bis 5 grösseren Wärmedurchgangskoeffizienten auf als die Schicht B. Mit anderen Worten: Beträgt der Wärmedurchgangskoeffizient der Schicht B beispielsweise 10 W/m²K, so beträgt der Wärmedurchgangskoeffizient der Schicht A bevorzugt zwischen 15 und 50 W/m²K.

In einer weiteren bevorzugten Ausführungsform weicht der Wärmedurchgangskoeffizient der Schicht C höchstens 50%, bevorzugt höchstens 30%, insbesondere höchstens 10%, vom Wärmedurchgangskoeffizienten der Schicht A ab.

In einer anderen bevorzugten Ausführungsform weicht der Wärmedurchgangskoeffizient der Schicht C höchstens 50%, bevorzugt höchstens 30%, insbesondere höchstens 10%, vom Wärmedurchgangskoeffizienten der Schicht B ab.

Erfindungsgemäss sind die U-Werte der Schichten A, B und C relativ, d.h. es spielt eine untergeordnete Rolle, welchen exakten U-Wert die einzelnen Schichten A, B und C der Schutzhülle (1) aufweisen, solange ein gewärmter passiver Wärmeträger (2a) Wärme durch die jeweilige Schicht so abgeben kann, dass beim Berühren der Schicht an der dem Wärmeträger (2a) gegenüberliegenden Schicht ein Wärmeempfinden auftritt, oder Wärme der berührenden Hautschicht durch die jeweilige Schicht der Schutzhülle (1) zum gekühlten passiven Kälteträger (2b) fliessen kann, dass beim Berühren der Schicht an der dem Kälteträger (2b) gegenüberliegenden Schicht ein Kälteempfinden auftritt.

Umfasst die Schutzhülle (1) eine Schicht C und ist diese an einer Seite der Schutzhülle (1) befestigt, kann die Schicht C einfach auf die andere Seite der Schutzhülle (1) gedreht werden. Dabei ist es oft von Vorteil, wenn die Schicht C zusätzlich noch an mindestens einer anderen Stelle reversibel fixiert werden kann, beispielsweise mit einem Befestigungsmittel (3), welches als Klettverschluss ausgebildet sein kann.

Umfasst die Schutzhülle (1) eine Schicht C und ist diese an zwei oder drei Seiten der Schutzhülle (1) zumindest teilweise befestigt, kann die Reihenfolge der Schichten A-B-C durch Umstülpen der Schutzhülle (1) auf einfache Art und Weise in B-C-A oder C-A-B geändert werden. Mit anderen Worten: Befindet sich der Wärme- oder Kälteträger (2) zwischen den Schichten A und B sowie die Schicht C neben der Schicht B (Reihenfolge: A-B-C), kann die Reihenfolge der Schichten durch Umstülpen der Schichten A/B und C so geändert werden, dass die Schicht C neben die Schicht A zu liegen kommt, welche durch den Wärme- oder Kälteträger (2) von der Schicht B getrennt ist (Reihenfolge C-A-B). Weist der Wärme- oder Kälteträger (2) eine gewisse Biegbarkeit auf, kann dieser problemlos in der Schutzhülle (1) gelassen werden, ohne dass er beim Umstülpen herausgenommen werden muss. Geeignete Wärme- und Kälteträger (2) mit genügender Biegbarkeit sind beispielsweise Fruchtkern-Kissen zum Wärmen und Gelkissen zum Kühlen. Und befindet sich der Wärme- oder Kälteträger (2) zwischen den Schichten B und C sowie die Schicht A neben der Schicht B (Reihenfolge: A-B-C), kann die Reihenfolge der Schichten durch Umstülpen der Schichten A und B/C so geändert werden, dass die Schicht A neben die Schicht C zu liegen kommt, welche durch den Wärme- oder Kälteträger (2) von der Schicht B getrennt ist (Reihenfolge B-C-A).

Umfasst die Schutzhülle (1) eine Schicht C in Form einer Lasche, ist diese typischerweise innerhalb der Schutzhülle (1) angeordnet und an einer Seite der Schicht A oder der Schicht B befestigt. Dabei kann die Schicht C, d.h. die Lasche, einfach von einer Seite des Wärme- oder Kälteträgers (2) auf die andere Seite gelegt werden. Ist diese zunächst zwischen der Schicht A und den Schichten C/B angeordnet, kommt durch Veränderung der Lasche der Wärme- oder Kälteträger (2) zwischen die Schicht B und die Schichtkombination A/C zu liegen.

In einer bevorzugten Ausführungsform besteht die Schicht A, die Schicht B und die optionale Schicht C der Schutzhülle (1) aus mindestens einem flächenförmigen, organischen Material, wobei die Materialien der Schichten A, B und C gleich oder verschieden sein können.

Vorteilhafterweise weist die Schicht B einen um einen Faktor 1.5 bis 5 kleineren Wärmedurchgangskoeffizienten auf als die Schicht A. So kann die Schicht B beispielsweise zwei (++) oder sogar dreimal (+++) so stark isolieren wie die Schicht A (+). Und die Schicht C kann beispielsweise den gleichen U-Wert wie die Schicht A oder die Schicht B aufweisen. Somit können die Schichten A, B und C beispielsweise aus dem gleichen Material bestehen, wobei die Schicht B aus zwei oder drei übereinander angeordneten Lagen der Schicht A hergestellt werden kann. Analoges gilt für die Schicht C, sofern sie nicht mit der Schicht A identisch ist.

Geeignete Materialien der Schichten A, B und/oder C der Schutzhülle (1) umfassen ein Textil, Gewebe, Vlies, Leder, Veloursleder, Kunstleder, Holz, Kork und/oder Plastik, wobei das Textil, Gewebe und/oder Vlies bevorzugt auf Naturfasern, insbesondere pflanzlichen Fasern, tierischen Fasern und/ oder mineralischen Fasern; und/oder Chemiefasern, insbesondere Fasern aus natürlichen Polymeren wie Cellulosefasern, Wollfasern und/oder Baumwollfasern, synthetischen Fasern wie Fasern aus Polyester, Polyamid, Nylon, Polyurethan und/oder Elastan, wobei die synthetischen Fasern auch in Form einer Mikrofaser vorliegen können, und/oder Fasern wie Carbonfasern auf anorganischen Baustoffen basieren können. Im Sinne der Erfindung gelten Fasern basierend auf anorganischen Baustoffen auch als organisches Material, sofern damit ein geeignetes flächenförmiges Material zur Herstellung der Schichten A, B und/oder C hergestellt werden kann.

Die zur Herstellung der Schichten A, B und/oder C eingesetzten Materialien - und somit auch die Schichten A, B und C wie auch die daraus hergestellte Schutzhülle (1) - sind vorteilhafterweise waschbar, beispielsweise bis 90°C, bevorzugt bis 60°C, insbesondere bis mindestens 30°C. Zudem eignen sich die Materialien für den Einsatz in Wäschetrockner wie Tumbler. Auch sind die aus den bevorzugt eingesetzten Materialien hergestellten Schichten A, B und C flexibel und biegbar, d.h. die einander gegenüberliegenden Ecken einer 20x20 cm grossen Schicht lassen sich ohne grossen Aufwand so biegen, dass sie sich berühren.

Die Schichten A, B und/oder C der Schutzhülle (1) weisen bevorzugt eine Materialdicke - beispielsweise eine Textildicke - von 20 bis 500 g/m², bevorzugt von 50 - 350 g/m² auf.

Die Schicht A, die Schicht B und die optionale Schicht C der Schutzhülle (1) sind bevorzugt mit einem Befestigungsmittel (3), insbesondere mittels Klettverschluss, Reissverschluss, Knöpfen, Druckknöpfen, Vernähen, Schnüren, Schweissen, Metallband und/oder Zip-Druckverschluss, welcher typischerweise aus Kunststoff hergestellt ist, zumindest teilweise aneinander befestigt oder können - typischerweise reversibel - aneinander befestigt werden.

In einer bevorzugten Ausführungsform der Schutzhülle (1) umfasst mindestens eine der Schichten A, B und/oder C eine weitere Schicht D, wobei die Schicht D an der Schicht A, B und/oder C angebracht ist und einen Latentwärmespeicher und/oder eine Isolationsschicht, insbesondere ein Isolationsvlies, eine Isolationsfolie, eine Aluminium-Isolationsfolie und/ oder eine Luftpolsterfolie, umfasst. Dabei kann der Latentwärmespeicher auf einem Paraffin basieren mit beispielsweise einem Schmelztemperaturbereich zum Wärmen von ca. 45°C oder zum Kühlen von ca. 0°C. Die weitere Schicht D kann auf der Schicht A, B und/oder C - reversibel oder irreversible - angebracht sein und/oder in mindestens eine der Schichten eingearbeitet sein. Wird an mindestens zwei Schichten eine Schicht D angebracht, können die Schichten D gleich oder unterschiedlich sein.

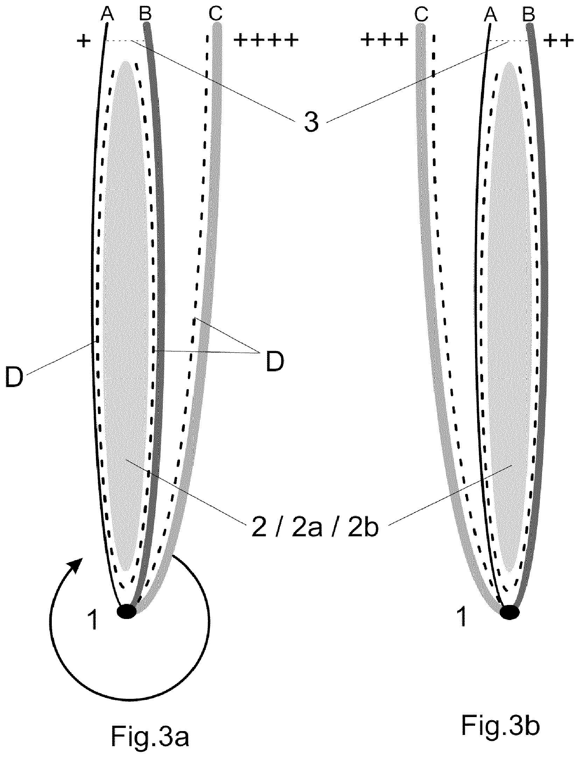

Die weitere Schicht D erhöht den U-Wert der Schichten der Schutzhülle (1) an die Schicht D angrenzenden Schicht A, B resp. C. Dadurch kann der gewärmte Wärmeträger (2a) eine höhere Temperatur und der gekühlte Kälteträger (2) eine niedrigere Temperatur aufweisen, wodurch ein länger anhaltendes Wärmen resp. Kühlen erzielt werden kann, ohne dem Patienten Schmerzen zu bereiten. Eine solche Ausführungsform umfassend die weitere Schicht D ist in Fig. 3a und 3b dargestellt.

Umfasst die Schicht D einen Latentwärmespeicher, kann die Schutzhülle (1) selbst gewärmt oder gekühlt werden. Dies erhöht die Zeit, während welcher die Schutzhülle (1) mit dem Wärme- oder Kältespeicher (2) eingesetzt werden kann. Zudem erlaubt diese Ausführungsform auch, dass die Schutzhülle (1) ohne einen Wärme- oder Kältespeicher (2) eingesetzt werden kann.

### Das Verfahren

Das erfindungsgemässe Verfahren umfasst auch die Schutzhülle (1) zur Anwendung im genannten Verfahren.

Das erfindungsgemässe Verfahren zum Wärmen oder zum Kühlen von Substraten mit der erfindungsgemässen Schutzhülle (1) umfasst die Schritte
a) mindestens ein gewärmter passiver Wärmeträger (2a) oder ein gekühlter passiver Kälteträger (2b) wird zwischen die Schicht A und die Schicht B oder zwischen die Schicht B und die optionale Schicht C der Schutzhülle (1) gelegt. Dadurch wird der in der Schutzhülle (1) angeordnete Wärme- oder Kälteträger (2) auf einer Seite mit einer Schicht oder mindestens zwei Schichten mit insgesamt einem grösseren Wärmedurchgangskoeffizienten und auf der anderen Seite mit einer Schicht oder mindestens zwei Schichten mit insgesamt einem geringerem, d.h. kleinerem, Wärmedurchgangskoeffizienten bedeckt, wobei die optionale weitere Schicht D bevorzugt in mindestens eine der Schichten A, B oder C eingearbeitet ist oder zwischen mindestens einer der Schichten A, B oder C und den Wärme- oder Kälteträger (2) zu liegen kommt.
b) die Schutzhülle (1) mit dem passiven Wärme- oder Kälteträger (2) so auf ein Substrat gelegt wird, dass die Schicht oder Schichten mit dem insgesamt geringeren Wärmedurchgangskoeffizienten der Schutzhülle (1) auf das Substrat zu liegen kommt,
c) die Schutzhülle (1) mit dem Wärme- oder Kälteträger (2) eine Zeit lang auf dem Substrat belassen wird bis die Wärmwirkung des Wärmeträgers (2a) oder die Kühlwirkung des Kälteträgers (2b) nachlässt,
d) anschliessend die Schutzhülle (1) mit dem Wärme- oder Kälteträger (2) gewendet wird, dass die Schicht oder Schichten der Schutzhülle (1) mit dem insgesamt grösseren Wärmedurchgangskoeffizienten auf das Substrat zu liegen kommt, und
e) die Schutzhülle (1) mit dem Wärme- oder Kälteträger (2) eine Zeit lang auf dem Substrat belassen wird bis die Wärmwirkung des Wärmeträgers (2a) oder die Kühlwirkung des Kälteträgers (2b) weiter nachlässt.

Dabei kann das Substrat, auf welches die Schutzhülle (1) mit dem Wärme- oder Kälteträger (2) gelegt wird, gegebenenfalls auch bedeckt sein, beispielsweise mit Verpackungsmaterial, einem Kleidungsstück oder einem Tuch.

Somit umfasst der Begriff die "Schutzhülle (1) ... auf das Substrat zu liegen kommt" immer auch, dass die Schutzhülle (1) auf ein bedecktes Substrat zu liegen kommen kann.

Der Begriff "Substrat" steht für ein zu behandelndes Material. Bevorzugte Substrate zur Behandlung mit einem Wärmeträger (2a) umfassen gewärmte Fertigspeisen für den Transport, beispielsweise für die Lieferung von einem Hersteller zu einem Kunden. Nicht-limitierende Beispiele umfassen online, Pizza und Takeaway Lieferdienste. Bevorzugte Substrate zur Behandlung mit einem Kälteträger (2b) umfassen Lebensmittel wie Tiefkühl-, Milch- und Fleischprodukte sowie Früchte. Bevorzugte Substrate zur Behandlung mit einem Wärme- oder Kälteträger (2) umfassen Körperstellen von Menschen und Tieren, wobei die Körperstellen auch bedeckt sein können, beispielsweise mit einem Kleidungsstück oder einem Tuch. Nicht-limitierende Beispiele umfassen im Aussenbereich Kleinkinder im Kinderwagen und ältere Personen beim Sitzen oder im Rollstuhl, wobei in der kalten Jahreszeit ein Wärmeträger (2a) und in der warmen Jahreszeit ein Kälteträger (2b) eingesetzt werden kann. Dabei wird der Wärme- oder Kälteträger (2) bevorzugt nicht direkt auf die nackte Haut aufgebracht, sondern auf Kleidungsstücke. Im Innenbereich wird die Schutzhülle (1) mit einem Wärmeträger (2a) bevorzugt zur Wärmebehandlung bei einer Schmerztherapie, bei Fieber, Grippe, verspannten Muskeln, Unterkühlung, Physiotherapie, Rehabilitation von Patienten nach Unfällen, Operationen und/oder Krankheit, zum Wärmen von Babys und Kleinkindern, beispielsweise auf Säuglingsstationen und in Kinderwagen und/oder zum Wohlfühlen, beispielsweise an kalten Wintertagen, eingesetzt. Und die Schutzhülle (1) mit einem Kälteträger (2b) wird bevorzugt bei Verbrennungen, Verbrühungen, Verletzungen wie beispielsweise Sportverletzungen, Entzündungen, Überhitzungen und/oder bei Kopfschmerzen eingesetzt.

Der Begriff "eine Zeit lang" ist eine relativer Begriff, wobei die effektive Zeitdauer abhängig ist i) von der Temperaturdifferenz des Wärme- oder Kälteträger (2) zum Substrat, auf welches die Schutzhülle (1) gelegt wird, ii) vom Wärmedurchgangskoeffizienten der Schicht oder Schichten, die zwischen dem Wärme- oder Kälteträger (2) und dem Substrat angeordnet sind, und iii) ob die Schutzhülle (1) mit dem Wärme- oder Kälteträger (2) direkt auf das Substrat oder auf eine das Substrat bedeckende weitere Schicht, beispielsweise ein Kleidungsstück, gelegt wird. Eine typische und bevorzugte "Zeit lang", d.h. Zeitdauer, beträgt mindestens 15, insbesondere mindestens 30 Sekunden, und kann bis 15 Minuten oder länger dauern.

In einer bevorzugten ersten Ausführungsform des Verfahrens umfasst die Schutzhülle (1) die Schicht C, wobei
- der Wärme- oder Kälteträger (2) zwischen die Schicht A und die Schicht B der Schutzhülle (1) gelegt wird und die Schicht C neben der Schicht B angeordnet wird, wobei die Schutzhülle (1) so auf das Substrat - oder deren Bedeckung - aufgebracht wird, dass die Schichten B/C - mit insgesamt kleinerem U-Wert - auf das Substrat zu liegen kommen,
- wenn die Wärmwirkung des Wärmeträgers (2a) oder die Kühlwirkung des Kälteträgers (2b) nachlässt, die Schicht C gedreht wird - beispielsweise durch umstülpen oder umklappen - sodass sie auf die Schicht A zu liegen kommt, wobei anschliessend die Schutzhülle (1) so auf das Substrat gelegt wird, dass die Schichten A/C auf das Substrat zu liegen kommen,
- bei weiterem Nachlassen der Wärmwirkung des Wärmeträgers (2a) oder der Kühlwirkung des Kälteträgers (2b) die Schutzhülle (1) mit dem darin liegenden Wärme- oder Kälteträger (2) gewendet wird, sodass die Schicht B auf das Substrat zu liegen kommt,
- bei weiterem Nachlassen der Wärmwirkung des Wärmeträgers (2a) oder der Kühlwirkung des Kälteträgers (2b) die Schicht C erneut gedreht wird, sodass sie wieder auf die Schicht B zu liegen kommt, wobei anschliessend die Schutzhülle (1) so auf das Substrat gelegt wird, dass die Schicht A auf das Substrat zu liegen kommt.

Der Begriff "Nachlassen der Wärmwirkung oder der Kühlwirkung" ist ein relativer Begriff und steht für subjektiv empfundenes Nachlassen der Wärmwirkung resp. der Kühlwirkung und kann von Fall zu Fall stark unterschiedlich sein. Oft wird ein Nachlassen der Wärmwirkung resp. der Kühlwirkung dann empfunden, wenn sich die Temperatur des Wärme- oder Kälteträgers (2) um 2°C, bevorzugt um 5°C, insbesondere um 10°C, der Temperatur der Substrate angleicht, auf welche die Schutzhülle (1) mit dem Wärme- oder Kälteträger (2) gelegt wird.

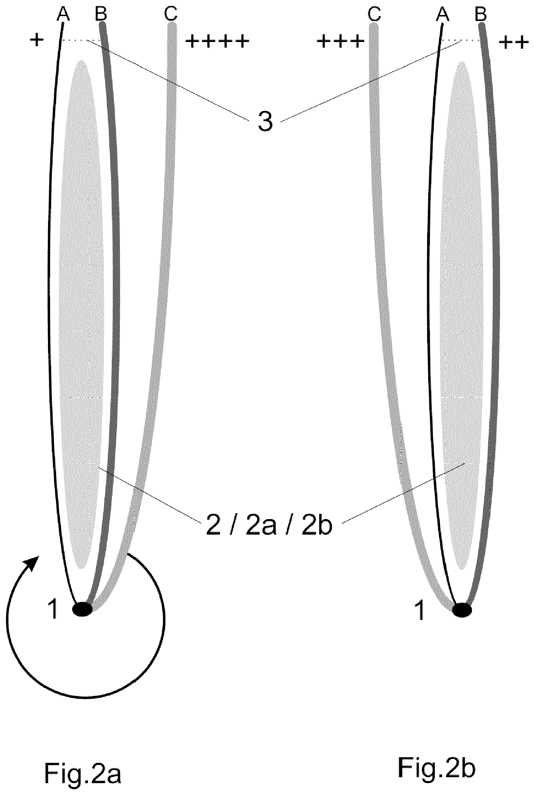

In dieser einen Ausführungsform, wie auch in Fig. 2a und Fig. 2b dargestellt, weist die Schicht C bevorzugt den gleichen oder einen ähnlichen U-Wert auf wie die Schicht B. Zudem kann die Schicht C nur an einer Seite mit den Schichten A und B verbunden sein und somit problemlos um diese Seite gewendet, d.h. umgeklappt resp. gedreht, werden, sodass sie abwechselnd neben der Schicht B (Fig. 2a) und der Schicht A (Fig. 2b) liegen kann. Mit anderen Worten: Befindet sich der passive Wärme- oder Kälteträger (2) zwischen den Schichten A und B der Schutzhülle (1), und ist die Schicht C neben der Schicht B angeordnet, weisen die beiden Schichten B und C einen noch kleineren U-Wert auf als die Schicht B allein. Kühlt sich der Wärmeträger (2a) ab, resp. erwärmt sich der Kälteträger (2b), kann die Schicht C umgeklappt werden, sodass sie auf die Schicht A zu liegen kommt. Je nach U-Wert der Schicht C kann der Patient die Schicht B oder die kombinierten Schichten A/C auf die zu behandelnde Stelle legen. Nach weiterem Kühlen/Erwärmen des Wärme- oder Kälteträgers kann beispielsweise die Schutzhülle (1) einfach gedreht werden, um die Oberfläche der Schutzhülle (1) mit der nächst geringeren Wärmedämmung auf die zu behandelnde Stelle zu legen. Und bei einem nächsten Schritt kann die Schicht C wieder zurück auf die Schicht B gedreht und die Schicht A mit dem grössten U-Wert auf die zu behandelnde Stelle gelegt werden. Somit weist die Schutzhülle (1) mit den drei Schichten A, B und C auf einfache Art und Weise vier verschiedene Wärmedurchgangskoeffizienten und somit vier verschieden stark isolierende Schichten auf.

In einer Alternative der einen Ausführungsform ist die Schicht C an mindestens zwei, bevorzugt an drei Seiten zumindest teilweise mit den Schichten A und B verbunden. Dabei basieren die Schichten A, B und C bevorzugterweise auf einem flexiblen, leicht verformbaren Material. Denn dadurch lässt sich die durch die Schichten B und C begrenzte Kammer der Schutzhülle (1) auf einfache Art und Weise von innen nach aussen stülpen, wodurch die Schicht C neu neben die Schicht A zu liegen kommt. Dabei kann der Wärme- oder Kälteträger (2) zwischen den Schichten A und B bleiben, wobei es vorteilhaft ist, wenn der Wärme- oder Kälteträger (2) ebenfalls eine gewisse Biegbarkeit aufweist. Durch das Umstülpen ist es ebenfalls auf einfache Art und Weise möglich, mittels den drei Schichten A, B und C vier verschieden Wärme-isolierende Schichtkombinationen der Schutzhülle (1) zu erhalten.

In einer anderen bevorzugten Ausführungsform des Verfahrens umfasst die Schutzhülle (1) die Schicht C, wobei das Verfahren folgende Schritte umfasst:
- Legen der Wärme- oder Kälteträger (2) zwischen die Schicht B und die Schicht C der Schutzhülle (1), wobei die Schicht A neben der Schicht B angeordnet wird und die Schutzhülle (1) so auf das Substrat gelegt wird, dass die Schichten A/B auf das Substrat - oder deren Bedeckung - zu liegen kommen,
- wenn die Wärmwirkung des Wärmeträgers (2a) oder die Kühlwirkung des Kälteträgers (2b) nachlässt, Wenden der Schutzhülle (1) mit dem darin liegenden Wärme- oder Kälteträger (2), sodass die Schicht C auf das Substrat zu liegen kommt,
- bei weiterem Nachlassen der Wärmwirkung des Wärmeträgers (2a) oder der Kühlwirkung des Kälteträgers (2b), Anordnen des Wärme- oder Kälteträgers (2), welcher zunächst zwischen der Schicht B und der Schicht C liegt, neu zwischen die Schicht A und die Schicht B, wobei anschliessend die Schutzhülle (1) so auf das Substrat gelegt wird, dass die Schicht A auf das Substrat zu liegen kommt.

Diese andere Verfahrensvariante kann auch einen weiteren vorgängigen Schritt umfassen, bei welchem der Wärme- oder Kälteträger (2) zunächst zwischen die Schicht A und die Schicht B der Schutzhülle (1) gelegt wird, wobei die Schicht C neben der Schicht B angeordnet wird und die Schutzhülle (1) so auf das Substrat aufgebracht wird, dass die Schichten B/C auf das Substrat - oder deren Bedeckung - zu liegen kommen. Wenn die Wärmwirkung des Wärmeträgers (2a) oder die Kühlwirkung des Kälteträgers (2b) nachlässt, wird der Wärme- oder Kälteträger (2) neu zwischen die Schicht B und die Schicht C gelegt. Anschliessend wird, wie oben beschrieben, die Schutzhülle (1) so auf das zu wärmende oder kühlende Substrat gelegt, dass die Schichten A/B auf das Substrat - oder deren Bedeckung - zu liegen kommen.

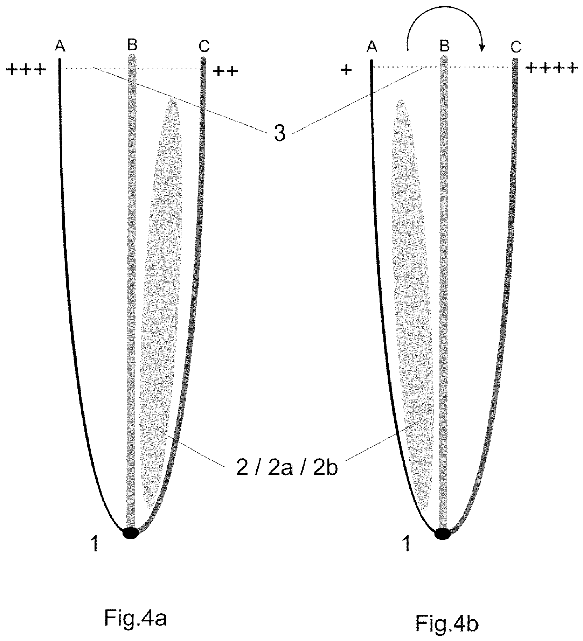

In einer Alternative der anderen Ausführungsform, wie in Fig 4a und 4b, dargestellt, ist die Schicht C an mindestens zwei, insbesondere an drei Seiten zumindest teilweise mit den Schichten A und B verbunden, wodurch zwei nebeneinander angeordnete Kammern entstehen, die durch die Schichten A und B respektive durch die Schichten B und C gebildet werden, wobei der Wärme- oder Kälteträger (2) einfach von einer Kammer zur anderen gewechselt werden kann. Dadurch ändert sich der U-Wert der Schicht und der Schichtkombination, zwischen welchen der Wärme- oder Kälteträger (2) angeordnet ist.

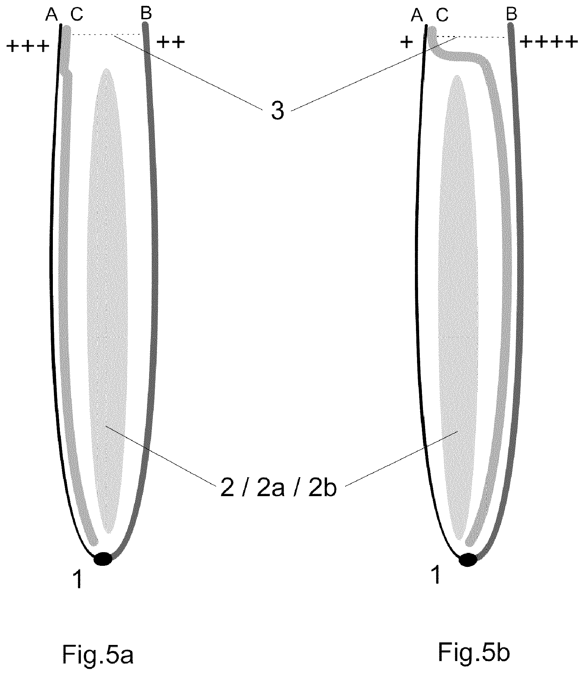

In einer weiteren Alternative der anderen Ausführungsform, wie in Fig 5a, 5b, dargestellt, ist die Schicht A und die Schicht B an mindestens zwei, insbesondere an drei Seiten zumindest teilweise miteinander verbunden und bilden zusammen eine Kammer. Die Schicht C ist typischerweise an einer Innenseite der Schicht A oder B in Form einer flexiblen Lasche im Bereich der Öffnung der Schutzhülle (1) oder an einer daran angrenzenden Seite befestigt. Um die Wärme-isolierende Wirkung einer Schicht (beispielsweise die Schicht B in Fig. 5a und Schicht A in Fig. 5b) oder Schichtkombination (beispielsweise die Schichten A/C in Fig. 5a und die Schichtkombination B/C in Fig. 5b) zu ändern, kann einfach die Lasche, d.h. die Schicht C, auf die andere Seite des Wärme- oder Kälteträgers (2) gelegt werden. Somit wird nicht der Wärme- oder Kälteträger (2) umplatziert, sondern die Schicht C wird von einer Seite zur anderen gewechselt.

### Die Verwendung

Die erfindungsgemässe Verwendung umfasst auch die Schutzhülle (1) zur Anwendung in der genannten Verwendung.

Die erfindungsgemässe Verwendung der erfindungsgemässen und erfindungsgemäss hergestellten Schutzhülle (1) umfasst die Wärme- und/oder Kältebehandlung von Substraten.

Zur erfindungsgemässen Verwendung bevorzugte Substrate umfassen Lebensmittel, insbesondere Tiefkühl-, Milch- und Fleischprodukte sowie Früchte, und/oder Körperstellen von lebenden Körpern von Menschen oder Tieren.

Die Wärme- und/oder Kältebehandlung von Körperstellen wird bevorzugt in Spitälern, medizinischen Praxen und/oder im privaten Bereich durchgeführt, wobei die Körperstellen auch bedeckt sein können, insbesondere mit einem Kleidungsstück oder einem Tuch.

Die Wärmebehandlung von Lebensmitteln wird bevorzugt eingesetzt bei gewärmten Fertigspeisen für den Transport, beispielsweise für die Lieferung von einem Hersteller zu einem Kunden. Nicht-limitierende Beispiele umfassen online-, Pizza- und Takeaway-Lieferdienste.

Die Wärmebehandlung von Körperstellen wird bevorzugt eingesetzt bei einer Schmerztherapie, bei Fieber, Grippe, verspannten Muskeln, Unterkühlung, Physiotherapie, Rehabilitation von Patienten nach Unfällen, Operationen und/oder Krankheit, zum Wärmen von Babys und Kleinkindern, beispielsweise auf Säuglingsstationen und in Kinderwagen und/ oder zum Wohlfühlen, beispielsweise an kalten Wintertagen.

Die Kältebehandlung von Lebensmitteln wird bevorzugt eingesetzt bei Tiefkühl-, Milch- und Fleischprodukten sowie bei Früchten.

Die Kältebehandlung von Körperstellen wird bevorzugt eingesetzt bei Verbrennungen, Verbrühungen, Verletzungen wie beispielsweise Sportverletzungen, Entzündungen, Überhitzungen und/oder bei Kopfschmerzen.

Zudem wird die Schutzhülle (1) bevorzugt im Aussenbereich zur Behandlung mit einem Wärme- oder Kälteträger (2) von Kleinkinder im Kinderwagen und ältere Personen beim Sitzen oder im Rollstuhl verwendet, wobei in der kalten Jahreszeit ein Wärmeträger (2a) und in der warmen Jahreszeit ein Kälteträger (2b) eingesetzt werden kann. Dabei wird der Wärme- oder Kälteträger (2) bevorzugt nicht direkt auf die nackte Haut aufgebracht, sondern auf Kleidungsstücke.

Im Folgenden werden nicht-limitierende, bevorzugte Ausführungsformen der erfindungsgemässen Schutzhülle (1) anhand der nachfolgenden Zeichnungen beschrieben. Diese sind nicht einschränkend auszulegen und werden als Bestandteil der Beschreibung verstanden:

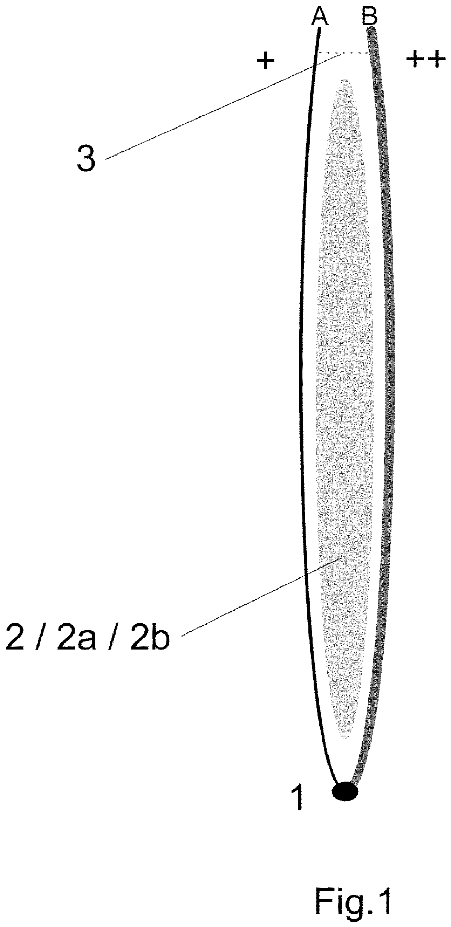

Fig. 1 zeigt beispielhaft die erfindungsgemässe Schutzhülle (1) mit einer Schicht A und einer Schicht B, wobei die Schicht A einen grösseren Wärmedurchgangskoeffizient (U-Wert) aufweist als die Schicht B. Somit weist die Schicht A eine geringere, d.h. kleinere,
Wärme/Kälte-isolierende Wirkung auf und fühlt sich dadurch wärmer an als die Schicht B. Dies ist symbolhaft mit + bei der Schicht A (schlechter isolierend) und mit ++ bei der Schicht B (besser isolierend) dargestellt.
   Zwischen den Schichten A und B ist der passive Wärme- oder Kälteträger (2) in Form eines gewärmten passiven Wärmeträgers (2a) oder eines gekühlten passiven Kälteträgers (2b) angeordnet. Im oberen Bereich der Schutzhülle (1) befindet sich beispielhaft ein Befestigungsmittel (3), mit welchem die vierte Seite der Schutzhülle (1) - durch welche der Wärme- oder Kälteträger (2) hineingeschoben werden kann - reversibel verschlossen werden kann, damit der Wärme- oder Kälteträger (2) nicht ausversehen heraus rutscht und die Haut schädigen kann.
Fig. 2a und 2b zeigen beispielhaft die Schutzhülle (1) mit der Schicht A, der Schicht B und der optionalen Schicht C. Der Wärme- oder Kälteträger (2) in Form eines gewärmten passiven Wärmeträgers (2a) oder eines gekühlten passiven Kälteträgers (2b) ist zwischen den Schichten A und B angeordnet (Fig. 2a), welche mit einem Befestigungsmittel (3) auch an der sonst offenen Seite miteinander befestigt werden können.
   Die Schicht A weist beispielhaft einen doppelt so grossen U-Wert auf wie die Schicht B. Der U-Wert der Schicht C entspricht in dieser Anordnung etwa demjenigen der Schicht B. Somit isolieren die nebeneinander liegenden Schichten B und C etwa viermal (++++) so fest wie die Schicht A (+). Dadurch isolieren die beiden Schichten B und C einen heissen Wärmeträger (2a) oder einen kalten Kälteträger (2b) wesentlich stärker als nur die Schicht A. Demzufolge kann der Wärmeträger (2a) resp. der Kälteträger (2b) eine grosse Temperaturdifferenz zum zu wärmenden oder zu kühlenden Objekt, d.h. Substrat, - typischerweise Lebensmittel oder eine Körperstelle - aufweisen, wenn die Schutzhülle (1) mit den Schichten B und C auf das zu behandelnde Substrat gelegt wird.
   Wird die Schutzhülle (1) mit dem Wärme- oder Kälteträger (2) mit den beiden Schichten B und C auf das Substrat gelegt, kann so die am meisten isolierende Wirkung erzielt werden.
   Nachdem die Wärmwirkung des Wärmeträgers (2a) oder die Kühlwirkung des Kälteträgers (2b) etwas nachgelassen hat, wird die Schicht C gedreht (Fig. 2b), sodass sie neben die Schicht A zu liegen kommt. Dadurch kommen die Schichten C und A nebeneinander zu liegenden isolieren somit etwa dreimal (+++) so fest wie die Schicht A alleine und die Schicht B isoliert etwa halb (++) so viel wie die vorherigen beiden Schichten B und C zusammen.
   Somit kann die Schutzhülle (1) mit dem Wärme- oder Kälteträger (2) mit den beiden Schichten C und A auf das Substrat gelegt werden, wodurch die isolierende Wirkung um eine Einheit abnimmt (von (++++) auf (+++)). Nach weiterem Nachlassen der Wärme- oder Kältewirkung wird die Schutzhülle (1) mit dem Wärme- oder Kälteträger (2) gedreht, sodass die Schicht B auf das Substrat zu liegen kommt, wodurch die isolierende Wirkung um eine weitere Einheit abnimmt (von (+++) auf (++)).
   Nach weiterem Nachlassen der Wärme- oder Kältewirkung des Wärme- oder Kälteträgers (2) wird die Schicht C erneut gedreht, sodass sie neben die Schicht B zu liegen kommt, wobei die frei werdende Schicht A auf das Substrat gelegt wird. Dadurch nimmt die isolierende Wirkung um nochmals eine weitere Einheit ab (von (++) auf (+)).
   Somit können mit der erfindungsgemässen Schutzhülle (1) umfassend einen gewärmter passiven Wärmeträger (2a) oder einen gekühlten passiven Kälteträger (2b) mit nur drei Schichten (Schicht A, B und C) auf einfache Art und Weise insgesamt vier verschiedene Wärme-/Kältestufen eingestellt werden.
   Das gleiche Resultat wird erzielt, wenn die durch die Schichten B und C begrenzte Kammer der Schutzhülle (1) von innen nach aussen gestülpt wird, wodurch die Schicht C neu neben die Schicht A zu liegen kommt.
Fig. 3a und 3b zeigen die Schutzhülle (1) mit dem Wärme- oder Kälteträger (2) in Form eines gewärmten passiven Wärmeträgers (2a) oder eines gekühlten passiven Kälteträgers (2b) analog Fig. 2a und 2b. Dabei ist im Innenbereich der Schutzhülle (1) an den Schichten A, B und C je eine weitere optionale Schicht D angebracht, wobei die Schicht D ein Latentwärmespeicher, beispielsweise ein Paraffin, und/oder eine Isolationsschicht, beispielsweise ein Isolationsvlies, eine Aluminium-Isolationsfolie und/oder eine Luftpolsterfolie, umfasst. Dadurch kann der gewärmte Wärmeträger (2a) eine höhere Temperatur und der gekühlte Kälteträger (2) eine niedrigere Temperatur aufweisen, wodurch ein länger anhaltendes Wärmen resp. Kühlen erzielt werden kann, ohne dem Patienten Schmerzen zu bereiten. Alternativ kann die Schutzhülle (1) umfassend einen Latentwärmespeicher als weitere Schicht D selbst gewärmt oder gekühlt werden und gegebenenfalls ohne Wärme- oder Kälteträger verwendet werden.
Fig. 4a und 4b zeigen beispielhaft die Schutzhülle (1) mit zwei Kammern, welche durch die Schichten A und B sowie durch die Schichten B und C gebildet sind. Analog Fig. 2a und 2b weist die Schicht A beispielhaft einen doppelt so grossen U-Wert auf wie die Schicht B und der U-Wert der Schicht C entspricht etwa demjenigen der Schicht B.
   In Fig. 4a ist der Wärme- oder Kälteträger (2) in Form eines gewärmten passiven Wärmeträgers (2a) oder eines gekühlten passiven Kälteträgers (2b) zwischen den Schichten B und C angeordnet, welche mit einem Befestigungsmittel (3), beispielsweise in Form eines Klettverschlusses, auch an der sonst offenen Seite miteinander verbunden werden können. Somit isolieren die nebeneinander liegenden Schichten A und B etwa dreimal (+++) so viel und die Schicht C etwa doppelt so viel wie die Schicht A (+) allein.
   Ist die isolierende Wirkung der beiden Schichten A und B (+++) zu gering oder die isolierende Wirkung der Schicht C (++) zu stark, kann der Wärme- oder Kälteträger (2) einfach in die durch die Schichten A und B gebildete Kammer umgebettet werden, wodurch dieser auf einer Seite durch die schwach isolierende Schicht A (+) und auf der anderen Seite durch die stark isolierenden Schichten B und C (++++) begrenzt wird, was in Fig. 4b dargestellt ist.
Fig. 5a und 5b zeigen beispielhaft die Schutzhülle (1) welche durch die Schichten A und B gebildet wird. Im Bereich der Öffnung der Schutzhülle (1) ist die Schicht C an der Schicht A befestigt, wobei die Schicht C in Form einer flexiblen Lasche in die Schutzhülle (1) hineinreicht. Dabei weist die Schicht A beispielhaft einen doppelt so grossen U-Wert auf wie die Schicht C, welcher beispielhaft etwa demjenigen der Schicht B entspricht.
   Im Bereich der Öffnung der Schutzhülle (1) ist an beiden Schichten A und C ein Befestigungsmittel (3), beispielsweise ein Klettverschluss, angeordnet, um die beiden Schichten miteinander zu verbinden.
   Fig 5a zeigt die Schutzhülle (1) nachdem der Wärme- oder Kälteträger (2) durch die Öffnung in die Schutzhülle (1) gelegt wurde. Dadurch ist der Wärme- oder Kälteträger (2) auf der einen Seite durch die beiden Schichten A und C und auf der anderen Seite durch die Schicht B begrenzt. Ist die isolierende Wirkung der Schichten A und C (+++) zu gering oder die isolierende Wirkung der Schicht B (++) zu stark, wird die Lasche, d.h. die Schicht C, auf die andere Seite des Wärme- oder Kälteträgers (2) verschoben, wodurch dieser auf einer Seite durch die schwach isolierende Schicht A (+) und auf der anderen Seite durch die stark isolierenden Schichten B und C (++++) begrenzt wird, was in Fig. 5b dargestellt ist.

## Patentansprüche

1. Schutzhülle (1) für passive Wärme- und Kälteträger (2) umfassend mindestens eine Schicht A und eine Schicht B, wobei die Schichten A und B an mindestens 2 Seiten zumindest teilweise aneinander befestigt sind, **dadurch gekennzeichnet, dass** die Schicht A einen grösseren Wärmedurchgangskoeffizienten (U-Wert) aufweist als die Schicht B.

2. Schutzhülle (1) nach Anspruch 1, **dadurch gekennzeichnet, dass** die Schutzhülle (1) eine weitere Schicht C umfasst, wobei die Schicht C an mindestens einer Seite an der Schutzhülle (1) befestigt ist und die Schicht C bevorzugt einen Wärmedurchgangskoeffizienten aufweist, welcher mit dem Wärmedurchgangskoeffizienten der Schicht A oder dem Wärmedurchgangskoeffizienten der Schicht B identisch ist.

3. Schutzhülle (1) nach Anspruch 1 oder 2, **dadurch gekennzeichnet, dass** die Schicht A, die Schicht B und die optionale Schicht C aus mindestens einem flächenförmigen, organischen Material besteht, wobei die Materialien der Schichten A, B und C gleich oder verschieden sein können.

4. Schutzhülle (1) nach Anspruch 3, **dadurch gekennzeichnet, dass** das Material ein Textil, Gewebe, Vlies, Leder, Veloursleder, Kunstleder, Holz, Kork und/oder Plastik umfasst, wobei das Textil, Gewebe und/oder Vlies bevorzugt auf Naturfasern, insbesondere pflanzliche Fasern, tierische Fasern und/oder mineralische Fasern; und/oder Chemiefasern, insbesondere Fasern aus natürlichen Polymeren wie Cellulosefasern, Wollfasern und/oder Baumwollfasern, synthetischen Fasern wie Fasern aus Polyester, Polyamid, Nylon, Polyurethan und/oder Elastan, wobei die synthetischen Fasern auch in Form einer Mikrofaser vorliegen können, und/oder Fasern wie Carbonfasern auf anorganischen Baustoffen basieren können.

5. Schutzhülle (1) nach mindestens einem der Ansprüche 1 bis 4, **dadurch gekennzeichnet, dass** die Schicht A, die Schicht B und die optionale Schicht C mit einem Befestigungsmittel (3), insbesondere mittels Klettverschluss, Reissverschluss, Knöpfen, Druckknöpfen, Vernähen, Schnüren, Zip-Druckverschluss, Schweissen und/oder Metallband zumindest teilweise aneinander befestigt sind oder aneinander befestigt werden können.

6. Schutzhülle (1) nach mindestens einem der Ansprüche 1 bis 5, **dadurch gekennzeichnet, dass** mindestens eine der Schichten A, B und/oder C eine weitere Schicht D umfasst, wobei die Schicht D an der Schicht A, B und/oder C angebracht ist und einen Latentwärmespeicher, beispielsweise ein Paraffin, und/oder eine Isolationsschicht, insbesondere ein Isolationsvlies, eine Aluminium-Isolationsfolie und/ oder eine Luftpolsterfolie, umfasst.

7. Verfahren zum Wärmen oder zum Kühlen von Substraten mit der Schutzhülle (1) nach mindestens einem der Ansprüche 1 bis 6, **dadurch gekennzeichnet, dass**
- mindestens ein gewärmter passiver Wärmeträger (2a) oder ein gekühlter passiver Kälteträger (2b) zwischen die Schicht A und die Schicht B oder zwischen die Schicht B und die optionale Schicht C der Schutzhülle (1) gelegt wird, wodurch der in der Schutzhülle (1) angeordnete Wärme- oder Kälteträger (2) auf einer Seite mit einer Schicht oder mindestens zwei Schichten mit insgesamt einem grösseren Wärmedurchgangskoeffizienten und auf der anderen Seite mit einer Schicht oder mindestens zwei Schichten mit insgesamt einem geringerem Wärmedurchgangskoeffizienten bedeckt ist,
- die Schutzhülle (1) mit dem passiven Wärme- oder Kälteträger (2) so auf ein Substrat gelegt wird, dass die Schicht oder Schichten mit dem insgesamt geringeren Wärmedurchgangskoeffizienten der Schutzhülle (1) auf das Substrat zu liegen kommt,
- die Schutzhülle (1) mit dem Wärme- oder Kälteträger (2) eine Zeit lang auf dem Substrat belassen wird bis die Wärmwirkung des Wärmeträgers (2a) oder die Kühlwirkung des Kälteträgers (2b) nachlässt,
- anschliessend die Schutzhülle (1) mit dem Wärme- oder Kälteträger (2) gewendet wird, dass die Schicht oder Schichten der Schutzhülle (1) mit dem insgesamt grösseren Wärmedurchgangskoeffizienten auf das Substrat zu liegen kommt, und
- die Schutzhülle (1) mit dem Wärme- oder Kälteträger (2) eine Zeit lang auf dem Substrat belassen wird bis die Wärmwirkung des Wärmeträgers (2a) oder die Kühlwirkung des Kälteträgers (2b) weiter nachlässt,
wobei das Substrat, auf welches die Schutzhülle (1) mit dem Wärme- oder Kälteträger (2) gelegt wird, gegebenenfalls auch bedeckt sein kann, beispielsweise mit Verpackungsmaterial, einem Kleidungsstück oder einem Tuch.

8. Verfahren nach Anspruch 7, **dadurch gekennzeichnet, dass** die Schutzhülle (1) die Schicht C umfasst, wobei
- der Wärme- oder Kälteträger (2) zwischen die Schicht A und die Schicht B der Schutzhülle (1) gelegt wird und die Schicht C neben der Schicht B angeordnet wird, wobei die Schutzhülle (1) so auf das Substrat aufgebracht wird, dass die Schichten B/C auf das Substrat zu liegen kommen,
- wenn die Wärmwirkung des Wärmeträgers (2a) oder die Kühlwirkung des Kälteträgers (2b) nachlässt, die Schicht C gedreht wird, beispielsweise durch umstülpen oder umklappen, sodass sie auf die Schicht A zu liegen kommt, wobei anschliessend die Schutzhülle (1) so auf das Substrat gelegt wird, dass die Schichten A/C auf das Substrat zu liegen kommen,
- bei weiterem Nachlassen der Wärmwirkung des Wärmeträgers (2a) oder der Kühlwirkung des Kälteträgers (2b) die Schutzhülle (1) mit dem darin liegenden Wärme- oder Kälteträger (2) gewendet wird, sodass die Schicht B auf das Substrat zu liegen kommt,
- bei weiterem Nachlassen der Wärmwirkung des Wärmeträgers (2a) oder der Kühlwirkung des Kälteträgers (2b) die Schicht C erneut gedreht wird, sodass sie wieder auf die Schicht B zu liegen kommt, wobei anschliessend die Schutzhülle (1) so auf das Substrat gelegt wird, dass die Schicht A auf das Substrat zu liegen kommt.

9. Verfahren nach Anspruch 7, **dadurch gekennzeichnet, dass** die Schutzhülle (1) die Schicht C umfasst, umfassend die Schritte
- Legen der Wärme- oder Kälteträger (2) zwischen die Schicht B und die Schicht C der Schutzhülle (1), wobei die Schicht A neben der Schicht B angeordnet wird und die Schutzhülle (1) so auf das Substrat gelegt wird, dass die Schichten A/B auf das Substrat zu liegen kommen,
- wenn die Wärmwirkung des Wärmeträgers (2a) oder die Kühlwirkung des Kälteträgers (2b) nachlässt, Wenden der Schutzhülle (1) mit dem darin liegenden Wärme- oder Kälteträger (2), sodass die Schicht C auf das Substrat zu liegen kommt,
- bei weiterem Nachlassen der Wärmwirkung des Wärmeträgers (2a) oder der Kühlwirkung des Kälteträgers (2b), Anordnen des Wärme- oder Kälteträgers (2) zwischen die Schicht A und die Schicht B, wobei anschliessend die Schutzhülle (1) so auf das Substrat gelegt wird, dass die Schicht A auf das Substrat zu liegen kommt.

10. Verwendung der Schutzhülle (1) nach mindestens einem der Ansprüche 1 bis 6 und des Verfahrens nach mindestens einem der Ansprüche 7 bis 9 zur Wärme- und/oder Kältebehandlung von Substraten.

11. Verwendung nach Anspruch 10, **dadurch gekennzeichnet, dass** die Substrate Lebensmittel, insbesondere Tiefkühl-, Milch- und Fleischprodukte sowie Früchte, und/oder Körperstellen von lebenden Körpern von Menschen oder Tieren, umfassen.

12. Verwendung nach Anspruch 11, **dadurch gekennzeichnet, dass** die Wärme- und/oder Kältebehandlung von Körperstellen in Spitälern, medizinischen Praxen und/oder im privaten Bereich durchgeführt wird, wobei die Körperstellen auch bedeckt sein können.
